# EUROPEAN PATENT SPECIFICATION

(11) **EP 0 529 397 B1**
(45) Date of publication and mention of the grant of the patent: **19.07.1995**
(21) Application number: 92113648.7
(22) Date of filing: 14.12.1988
(51) Int. Cl.: C10G 9/00, G01N 33/28, G01N 15/14

(54) **A method for determining an instantaneous reference parameter of tar stability in a thermal conversion oil plant**
Verfahren zur Bestimmung des momentanen Referenzparameters für die Teerstabilität in einer thermischen Ölkonversionsanlage
Procédé pour déterminer un paramètre instantané de référence pour la stabilité des goudrons dans une installation de conversion thermique d'huile

(30) Priority: 16.12.1987 IT 4871987; 16.12.1987 IT 4872087
(43) Date of publication of application: 03.03.1993
(62) Divisional of application: 88830539.8
(73) Proprietor: CHIMEC S.P.A., I-00144 Roma RM (IT)
(72) Inventor: Faina, Roberto, I-00156 Roma RM (IT); Ingrassia, Mario, I-00124 Roma RM (IT); Zanotti, Andrea, I-00137 Roma RM (IT)
(74) Representative: Bazzichelli, Alfredo

(56) References cited:
- EP-A- 0 234 857
- FR-A- 2 578 979
- US-A- 3 952 580
- US-A- 4 444 044
- US-A- 4 619 756

## Description

The present invention relates to a method for determining an instantaneous reference parameter of tar stability in a thermal conversion operation to control the operating conditions in an oil treating plant.

A thermal conversion process is a process in which, by the action of temperature, large hydrocarbon molecules, and in particular those having a boiling temperature higher than 350°C, are split into smaller and lower boiling molecules. This type of process is currently used in the industry of crude oil refining for obtaining intermediate or light cuts of higher value from heavy residues of low commercial value.

These operations are carried out in refinery by plants such as visbreaking, coking, hydrocracking.

It is well known that the above mentioned plants involve an operation under particularly severe fouling conditions, particularly the so-called "visbreaking" plants, which in particular treat bottom products from upstream atmospheric (topping) or vaccuum distillation plants.

The fouling characteristics of the charge are in fact the controlling factor in these types of plant, in that on them depend the choice of the process temperature and finally the yield in light fractionation products, such as petrol and gasoil. A further controlling factor in these plants is the quality of the bottom residue obtained (tar), which should be provided with "stability" characteristics to be further used in downstream plants.

At present an acceptable level of the residue obtained at the bottom of the fractionating column is established, based on standard numerical indexes and the process temperature is fixed as a function of this parameter.

Visbreaking (VSB) in particular is gaining more and more importance in Europe and in industrial countries in view of the decrease in the fuel oil market and the expansion of consumption of middle distillates, in particular gasoil for transportation. The visbreaking plant (VSP) is schematically formed of the following parts. A train of exchangers in which a feed charge is fed for pre-heating, then a furnace in which the real thermal cracking is carried out, then a fractionating column from the bottom of which the tar is extracted which goes through the heat exchangers transferring part of its heat content to the charge. It is also possible to provide a "soaker" between the furnace and the fractionating column. The operating conditions of such thermal cracking plants are briefly the following: a charge comprising a distillation or vaccuum residue is introduced into a furnace operating at a temperature of about 420-500°C (in the case of presence or absence of "soaker" respectively) at a pressure between 3 and 20 bar. The charge thus treated passes to a fractionating column from which light distillates (about 3 to 10% weight), middle distillates (15 to 20% weight) and a residue (tar, 65 to 75% weight of the charge) are obtained. The plants are so sized as to provide such dwelling times at the cracking temperature as to carry out the conversions required in the process. By the term "conversion" is intended the light and middle distillate percentage (gas plus petrol plus gasoil) with respect to the total charge. Products thus obtained from the fractionating column have the following final destinations:
a) gas and GPL are usually introduced into the refinery network;
b) petrols (high octane generally) go to the petrol "pool" after hydrogenation and desulphuration;
c) gasoils, which are instable and show a net tendency to polimerization, are used as far as possible, after eventual hydrogenation, as components of finished gasoils or as fluxes; and
d) the residue (tar) present in a higher amount, is instable due to the presence of insaturated compounds and cracking side products (coke and asphaltenes) and is transformed into fuel oil.

The VSB process was developped some years ago with the intention of obtaining a viscosity decrease in heavy products. Today, however, it is managed with substantially different objects, namely with the aim of obtaining a maximum transformation into middle and light distillates to meet the market requirements.

It has to be noted that even a conversion increase by 1% in the treated charge has to be considered extremely satisfying in terms of profit.

The controlling factor in obtaining a high conversion is the need to obtain a stable residue. In fact an increase of the cracking temperature certainly would involve a higher conversion in light and middle distillates, but it would produce a much more instable tar which would require a higher addition of flux for its transformation into fuel oil.

The stability of gasoil and tar is measured as the tendency to separation of the polimeric and asphaltenic components from the paraffinic components in time. In fact the composition of such distillates comprises a paraffin or aliphatic portion and an asphaltene or essentially aromatic portion. Since asphaltenes, by definition, are macromolecular aggregates of naphthenic type with a low hydrogen content, insoluble in N-hexane or paraffins, the reasons for the instability can be clearly understood. The ratio of the paraffin portion to the naphthene portion is clearly influenced by the origin of the crude oil which is treated in the plant.

The conversion of middle distillates is increased, the charge being the same, either by increasing temperature or by increasing the reaction time, (however always effecting the tar stability). There is consequently a relation according to which the cracking temperature and the dwelling time are a function of the required tar stability.

From the foregoing it is self-evident how the need has been felt for tests able to define the stability of residues and thus to define the limit conditions for operating with a definite tar stability.

The tests known at the state of the art are empirical tests and are essentially as follows:
a) flocculation ratio (FR-5);
b) peptization index (PV=peptization value); and
c) further less used tests, such as "Martin Bailey".

These tests of empirical character as stated herein before, are essentially based on the capability of the paraffin fluids to cause asphaltene type substances to precipitate. Practically, in carrying out these tests, mixtures are used containing a paraffin hydrocarbon portion (ketane, isooctane) and an aromatic portion (essentially xylene) and the concentration is measured at which asphaltene precipitates or is solubilized, depending on the type of procedure employed, by modifying the composition of these mixtures.

The most widely used test at present for controlling the tar stability is the P-value (peptization index). This test also is based on the measure of instability of the asphaltene portion of tar, on varying the solvent concentration (aliphatic plus aromatic mixture) in which the tar is dissolved. Assuming 1 as an absolute limit of tar instability in plant operation, it is a good practice to operate at a PV value not lower than 1,05 and usually at a PV value of from 1,05 to 1,15, although higher values can be accepted.

Besides this test, as stated herein before, there are others such as HFT, ASTM D-1661, UOP 1174, which, however, are rarely used due to their complexity, scarce validity or length of response time. The Applicant has developed the determination of an additional index numeral, denominated Chimec Value (CV) which will be illustrated hereinafter.

The object of the invention is a method for determining the instantaneous reference parameter of tar stability defined as coke or fouling deposition tendency in a thermal conversion of oil treating plant, characterized by the following operations;
taking a drop of bottom residue obtained from the plant during operation with a determined furnace outlet temperature;
observing said drop under the microscope and counting the number of amorphous carbon particles having a size ranging from about 1 um up to 20 um, which are to be found within a pre-established area of the field of vision of the microscope; and
assuming said number of particles as instantaneous reference parameter of tar stability, to be compared with a standard index number chosen from a scale of reference index numbers determined in a blank test on each plant, for regulating the outlet temperature of the furnace to a higher or lower value, proportionate to the respectively negative or positive difference between said number of particles and the index number chosen for the running of the plant in pre-determined conditions.

In EP patent 0321424 it is described that a considerable increase in the yield of thermal conversion oil plants can be obtained by means of a process which in one of its steps provides adding a composition comprising alkylsuccinimides to the plant charge. It is important to note that the yield increase can be considered either an increase of conversion in middle and light distillates, the tar stability being the same, or simply an increase of the tar stability, the conversion into middle and light distillates being the same.

Alkylsuccinimides used in the composition to be added to the plant according to the process illustrated in EP 0321424 are described in DE-OS No. 2053800, British patents Nos. 960493 and 1186659 and in US Patent No. 3172892. In the above mentioned patents, however, there is no reference to the use of such substances in a process for increasing the yield in thermal conversion oil plants. Preferred representatives of these substances are: alkylsuccinimides having a total basicity value from 40 to 60 mg KOH/g and nitrogen content from 2,5 to 3,5% by weight.

It is important to note that this process can be used for producing a more stable tar and to this object it is no longer necessary to increase the cracking furnace temperature, the yield in this case being obviously due to the fact that the tar thus produced is more stable and it consequently requires a lower addition of fluxes in order to be further used successively.

The antioxidants to be added according to the process can be of phenol or amine type; preferred representatives are 2,6 di-tert-butyl-4-methylphenol, tributylamine, N,N-dimethyldodecilamine.

As metal deactivators to be used, particularly preferred is the following: N,N'-disaliciliden-1,2-diaminopropane.

The preferred dosage for the composition to be additioned into step b) of the process is in the range from 20 to 500 ppm referred to the feed charge. This addition can be made in the above indicated points and as a function of the yield increase to be obtained, as will be better illustrated hereinafter in the following examples.

### EXAMPLES

In the following examples, to better illustrate the process, the following additives have been used: additive A comprising alkylsuccinimide and usual solvents and additive B comprising alkylsuccinimide, an amine or phenol type antioxidant, and a metal deactivator and solvents.

The conversion yields depend both on the type of plant and the type of crude oil to be treated.

### Example 1 (Refinery D)

BASE SPECIMEN: charge: crude oil of type Iranian Heavy + Ural; rate: 20 ppm of additive A + 10 ppm of additive B; peptization value (PV) = 1,05; gasoil yield = 18,4% by weight.
SPECIMEN A: charge: same charge and same operating conditions; rate: 80 ppm additive A + 10 ppm additive B; PV = 1,1. A PV increase of 0,5 is obtained.
SPECIMEN B: same charge at a reaction temperature higher by 2°C; rate: 80 ppm additive A + 10 ppm additive B; PV = 1,05; gasoil yield = 20,9 % by weight. An increase of gasoil yield by 2,5% by weight is obtained.

As it can be noted from the above illustrated data, an increase of addition of the composition based on alkylsuccinimides according to the process of the present invention, enables the PV to be increased from 1,05 to 1,1, that practically means that the tar stability is increased, while the yield is maintained on the same level. It is moreover possible, even maintaining a PV at 1,05 (that is the limit value of tar stability recommended in these plants) to increase the yield by increasing the temperature by 2°C, thus obtaining as a consequence a higher gasoil yield by 2,5% by weight.

### Example 2 (Refinery H)

The plant is a visbreaking plant fed by an Essider type atmospheric residue.

The cracking conditions were intentionally made extreme for carrying out the test according to the process of the present invention.
1) Determination of the instantaneous reference parameter of tar stability (in this case a determination of PV was involved) made at 487°C; PV = 1,00 (condition of maximum instability).
2) Addition of product A (40 ppm) into the plant charge and product B (20 ppm) into the tar of the column bottom.
3) New PV determination of tar, which this time was 1,04.
4) Increase of the cracking temperature until the PV value was brought back to the initial one; this temperature increase was by 3°C.

An increase by 3°C with this type of crude oil has produced a considerable increase in yield of light distillates equal to 1,5% by weight of total charge.

### Example 3 (Refinery C)

Visbreaking plant integrated with vaccuum and thermal cracking fed by a Dubai type atmospheric residue.

The stability is determined on the tar product of the integrated plant (visbreaking tar + thermal tar + flux).
1) Initial PV at 480°C = 1,1.
2) Addition of 40 ppm additive A into the charging pump.
3) The new PV value of tar was 1,25.
4) To bring the PV back to 1,1, the cracking temperature was raised by 2° C with consequent increase of yield by 1,8% by weight referred to the total charge.

### Example 4 (Refinery I)

Visbreaking plant provided with soaker, fed by atmospheric residue (blending of Egyptian and Russian crude oils). In order to evaluate the tar stability in this case the FR test (flocculation ratio) was used.
1) Determination of the instantaneous reference parameter of tar instability; initial flocculation ratio = 68 at a cracking temperature of 452°C.
2) Addition of additive A (60 ppm) and additive B (40 ppm).
3) Determination of the new instantaneous reference parameter of stability in the plant; FR = 66.
4) In order to bring the instantaneous reference parameter of tar stability back to value 68, the cracking temperature was raised to 454°C with an increase of conversion by 2 points.

In this case an increase of the instantaneous reference parameter of tar stability FR corresponds to a higher tar instability in contrast to the case of a test based on PV.

It is important to note that in plants provided with soaker it is generally estimated that an increase in cracking temperature by 2°C corresponds to two points of higher conversion. This derives from the fact that, as previously stated, an increase of the dwelling time produces the same effect as an increase in cracking temperature.

### Example 5 (Refinery C)

In this refinery the action of the additive composition was evaluated for some days with different crude oil charges.

The additive was added upstream the furnace and samples of residue were taken from the preflash bottom for the determination of PV.

The refinery made usual routine tests by determining PV on a fluxed mixture formed of vaccuum residue (Visbreaking tar) and thermal residue, about 80:20%.

All tests were carried out maintaining the plant parameters constant, such as the flow rate (265 tons/hour), the type of charge, the cracking temperature during each addition of additive. The addition was maintained for about four hours, minimum time required to obtain a response in the residue, and it ranged from 40 to 60 ppm.

The results are referred in the following Table 1.

**TABLE 1**

| DAY | HOUR | ADDITIVE DOSE ppm | CHARGE & DENS. 20°C | TUF °C | P.V. FLUX | P.V. PREFLASH | HFT % | NO.PARTICLES >5» |
|---|---|---|---|---|---|---|---|---|
| 27/7 | 12,00 | 0 | OMAN ≈15% | 480 | 1.4 | 1.2 | ND | 70 |
| 27/7 | 16,00 | 58 | BELAYM 0.99 | 480 | --- | 1.3 | ND | 50 |
| 28/7 | 10,00 | 0 | OMAN ≈15% | 482 | 1.5 | 1.2 | 0.08 | 45 |
| 28/7 | 15,00 | 45 | BELAYM 0.99 | 482 | --- | 1.3 | 0.07 | 25 |
| 29/7 | 08,30 | 0 | OMAN | 482 | >1.4 | 1.1 | 0.058 | 60 |
| 29/7 | 13,30 | 45 | | 482 | >1.5 | 1.15 | 0.030 | v. fine |
| 30/7 | 09,00 | 0 | DUBAI 0.93 | 482 | 1.1 | 1.1 | 0.137 | 30 |
| 30/7 | 13,00 | 40 | 0.93 | 482 | --- | 1.25 | 0.110 | 10 |
| 30/7 | 21,00 | 40 | 0.93 | 482 | --- | 1.1 | 0.021 | 10 |

Table 1 resumes the data collected in the four days of tests, three with a Oman oil charge and the last day with Dubai crude oil.

The Table reports the PV measured on the preflash bottom and the routine PV measured on the fluxed mixture.

For each day a run without additive addition and a run with additive addition are reported by comparison, the conditions being the same.

From an examination of the data on the Table the following observations can be made.

The PV determination on the fluxed mixture was effected each morning by routine. Only in one case (29/7) it was effected after the addition, in that it was less significant with respect to that effected on the preflash bottom as such. In this case the observed increase was from 1,4 to 1,5.

The P value determined on the preflash bottom rose with the additive addition and, at the rate used, it was necessary to raise the cracking temperature by about 2°C to obtain the same PV again.

Similarly, a decrease in number and size of the coke agglomerates (number of particles larger than 5») is observed.

The decrease of these agglomerates is confirmed by variations of HFT (coal percentage the residue). In particular on day 30/7, the addition of additive produced a decrease of HFT from 0,137% to 0,021%.

### Example 6 (Refinery D)

Visbreaking plant integrated with direct draining topping without heat exchange train; reaction in a cathedral furnace; charges and blending of various middle east crude oils.

In this example determinations were effected following both the peptization value test and the flocculation ratio test.

The plant was treated with additive A (20 ppm) dosed in the charging pump.

Initial values: with 30 ppm additive A already present, flocculation ratio = 74; peptization value = 1,07, both measured at a temperature of 484°C. Increase of rate of additve A to 80 ppm.

The results of the new stability values were: FR = 56; PV = 1,15. To bring back the instantaneous reference parameters of tar stability to their initial values it was necessary to raise the cracking temperature by 4°C.

In the following Table 2 the data are globally reported referring to different plants present in the various refineries. The Table shows a comparison between an increase of the outlet furnace temperature (TUF) made possible by an additive A rate as ppm necessary for maintaining the same tar stability value prior to the use of additve A. It was possible in each case to determine which dose of additve was to be added to have a determined increase in the outlet furnace temperature on maintaining the same PV value, or by how much it is possible to increase the tar stability.

**TABLE 2**

| | **T.U.F.** | **P.V.** | **DOSE OF PRODUCT A (ppm)** |
|---|---|---|---|
| 1) REFINERY C | | 1,05 | 0 |
| | 482 | 1,07 | 20 |
| | 484 | 1,05 | 20 |
| 2) REFINERY H | | 1,00 | 0 |
| | 487 | 1,04 | 60 |
| | 490 | 1,00 | 60 |
| 2) REFINERY I | | 1,1 | 0 |
| | 480 | 1,2 | 40 |
| 1) REFINERY B | 468 | 1,1 | 0 |
| | 476 | 1,1 | 45 |
| 1) REFINERY A | 470 | 1,07 | 0 |
| | 473 | 1,07 | 45 |
| KEY: 1) = average data of run; 2) = punctual data. | | | |

In figure 1 a diagram is shown in which ppm of additive A are represented on the ordinate axis, and the Δ TUF increase, that is the increase of outlet furnace temperature which can be obtained by the addition of a fixed amount of product A ppm, is represented on the abscissa. The line having a higher angular coefficient (line 1) expresses the average of the most unfavourable conditions depending on the response by the product (or the products) and on the characteristics of the charge oil (content in asphaltenes and base of the oil). The other line (line 2) having a lower angular coefficient, expresses on the contrary the more favourable case. On the first line for obtaining a Δ TUF of 2°C it is necessary to add an amount of additive A of about 40 ppm. In the case of the line having a lower angular coefficient, that is in the best case, it is sufficient to add only 20 ppm. Within these two lines, anyway, can be found the amount of ppm to be added to obtain a determined Δ TUF, always maintaining, obviously, the same tar stability.

### Example 7 (Refinery Z)

In this example the refinery was run with the same type of charge for several days.

Object of the experiment was to verify the action of the additive on the tar stability, expressed as PV and CV and to find a temperature of higher conversion equivalent to the same conditions of tar stability in the absence of additive.

At the beginning of the experiment the plant was running at an outlet furnace temperature of 440°C with an additive rate of 20 ppm. The PV measured on the residue was 1,13.

The temperature was successively raised by 1°C, bringing the outlet furnace temperature to 441°C. This involved a decrease of PV, within still acceptable limits, but an increase in the plant yield.

The additive addition at a rate of 20 ppm was then interrupted for a certain number of hours. A decrease of PV and a corresponding increase of CV was observed, until the additive addition was reestablished at a rate of 80 ppm. In the succeeding hours the PV value to rise again, whereas the CV value was decreasing to a lower level.

The results are reported in detail in the following Table 3.

**TABLE 3**

| HOURS | TUF °C | ADDITIVE DOSE (ppm) | P.V. | C.V. | AVERAGE CONVERSION 24h (%) | ISOVISCOSITY AVERAGE YIELD 24h(%) |
|---|---|---|---|---|---|---|
| 0 | 440 | 20 | 1,13 | -- | 23,09 | 21,8 |
| 3 | 441 | 20 | -- | -- | | |
| 7 | 441 | 20 | 1,10 | 8-10 | | |
| 18 | 441 | 0 | -- | -- | | |
| 22 | 441 | 0 | 1,05 | 15 | | |
| 30 | 441 | 0 | 1,07 | 15 | 26,14 | 23,07 |
| 31 | 441 | 80 | -- | -- | | |
| 35 | 441 | 80 | 1,09 | 5-10 | | |
| 47 | 441 | 80 | 1,1 | <5 | 26,57 | 23,16 |

From Table 3 can be observed the higher efficiency in the determination of tar stability by means of Chimec Value, in comparison with P Value, though the results are consistent.

Table 3 also shows the average conversion percentage in 24 hours and the average yield percentage in 24 hours, referrred to the time in which the plant is stabilized in the different conditions.

It can be observed that the conversion and yield values were increased with respect to the condition at 0 hours, whereas the PV values were maintained within acceptable limits. In conclusion it can be observed that in the plant under test, an increase of PV obtainable with an additive rate of 80 ppm is about 0,06 points, with a conversion increase on a 24 hour average by about 3,4% and a yield increase on the 24 hours average by about 1,36%, due to a rise by 1°C of the outlet furnace temperature.

As already stated hereinbefore, the index values for an evaluation of the stability used in the prior art are mainly three: the P value or "PV", the flocculation ratio or "FR", and the xylene equivalent or "Martin Bailey" or "MB".

The above described three presently used tests all give more or less precise information on the stability of the residue, that is on the capability of asphaltenes to remain in a colloidal form and as a consequence not to precipitate from the system.

While on the one hand these tests ensure, apart from the uncertainty of measurement, the stability of the bottom residue obtained, they have no direct (proportional) relation to the fouling of a plant caused by coke, that is by the maximum dehydrogenation step of asphaltenes themselves.

Consequently, even if the plant is controlled on the basis of the above mentioned index values, it can become possible that the plant has to be stopped for a fouling excess before the expected time, with a consequent loss of economic yield in the refinery unit.

The Applicant has developped a different index value, hereinafter denominated Chimec Value (CV), by which not only the stability of the residue can be controlled, but a direct and proportional indication can be had of the fouling characteristics of the plant which is treating the charge resulting in the residue itself. As the fouling factor is by far more important commercially than the parameter of residue stability, the method according to the invention enables the plant to be operated at an optimum process temperature to obtain a bottom residue having an acceptable stability consistent with an acceptable fouling factor, depending on the inlet charge.

The method for the determination of CV comprises the following operations: sampling a drop of bottom residue as obtained from the plant in operation at a determined outlet furnace temperature; subjecting the drop to microscopic observation and counting the number of amorphous coal particles having a size ranging from about 1 »m to 20 »m, which are found within a predetermined area in the field of the microscope eyepiece; comparing this number of particles with a standard index numeral selected from a scale of reference index numerals indicating absolute fouling characteristics of the plant; and adjusting the outlet furnace temperature at an increased or decreased level, in proportion to the negative or positive difference respectively between this number of particles and the index numeral selected for the plant operation in prefixed fouling contitions.

The CV test, as a consequence, is based on a reading under the microscope of the number of amorphous coke particles and their sizes on a slide on which a drop of residue has been placed, that is on the same sample of tar obtained from a visbreaking, of which the tests of stability are effected according to the prior art tests.

The basic apparatus for carrying out the test comprises an optical microscope with an eyepiece typically of minimum 100 enlargements and slides and covers for microscope preferably of 4cm².

To effect the measurement, the tar sample is homogenized, eventually heating in the case it is highly viscous at a maximum temperature of 70°C. A drop of 4»l is taken and placed on a slide, the drop is covered with a square cover and is slightly pressed to spread it on the whole square, thus obtaining a depth of about 10»m.

Putting the slide in the microscope the dark point-shaped particles are counted. The counting can be standardized by establishing a given counting field. This field can be a reticulation on the eyepiece, or the whole ocular field by placing this at the precise center of the square cover.

To avoid subjective errors, the slide can also be shifted under the eyepiece, so as to count all the particles which successively appear in the eyepiece field. This avoids errors due to counting of impurities present on the eyepiece, which remain fixed, while the particles are shifted with the slide.

It is well known from the literature that there are a number of aggregation states of carbon, however an amorphous form and a more crystalline form of graphitic type can be basically distinguished. These two forms are distinguished by the ratio H/C which is lower in the case of crystalline particles.

With respect to the CV test it has been found that the count has to be effected on the amorphous carbon particles so that the needle-shaped particles of graphitic type are neglected.

Among the amorphous particles, only those having a size between 1»m and 20»m are counted, in that particles larger than 20»m are not significant to the purpose of the evaluation in question, and particles smaller than 1»m are not relevant as far as the plant fouling is concerned.

The number of particles thus counted represents the index numeral which, when compared to a preset scale of index numerals, which express a fouling characteristic of the plant in an absolute sense, gives an indication for controlling the process temperature.

As an example, with cover glasses 2 cm² and a standard particle count by the procedure of shifting a 2cm long glass underneath the microscope, it has been found that a particle number lower than 70 means an insignificant fouling condition, a particle number from 70 to 130 indicates a mild fouling condition, a count between 130 and 200 indicates a considerable fouling, and a count higher than 200 indicates conditions of excess fouling.

When, on the contrary, the reading is effected at the center of the sample (surface about 3 mm² at 100 enlargements) it has been found that: a count from 0 to 10 particles indicates an insignificant fouling; from 11 to 25 particles a mild fouling; from 26 to 50 particles a considerable fouling; above 50 particles an excess fouling.

In parallel to this, the same index numerals indicate the fouling factor in the plant operation with a predetermined charge, so that it is possible to previously establish the period of plant operation, maintaining the index on the tar residue constant.

The CV test enables a more precise evaluation to be made of the coke agglomerates which cause fouling and the possible inhibition of this effect by action of a dispersing product. In this latter case, in fact, the size and number of particles will be certainly lower.

An apparatus for carrying out the CV test basicly comprises a microscope with an optical system from 20 to 500 enlargements, preferably 100, which can be improved or made in part or totally automatic, by connecting a photographic apparatus to the microscope to effect a more precise counting on a picture, or by connecting a telecamera to the microscope, which transmits enlarged images, preferably to a monitor or display, or by connecting a system of this type with an electronic processor being able to manage through a specific software the automation of the counting and distribution of the particle sizes. The computerized system can be connected to a system controlling the operating parameters of the plant, such as in particular the outlet furnace temperature, in order to control the parameters as a function of the counting thus made. This system can be inserted in the plant so as to enable a continuous evaluation of the coke size by comparing this with the limit values in real time, to enable an immediate optimization of the process parameters to be made.

In the following some effective examples are referred which compare the Chimec Value with conventional indexes.

### Example 8 (Refinery I)

This refinery was operating on a charge consisting of a mixture of middle-east oils at a rate of low conversion and in these conditions a xylene equivalent value (MB) of 48 was recorded, whereas the day after the MB value changed to 66, on raising the outlet furnace temperature from 438 to 454°C.

In a side-by-side way the particle count was carried out at the microscope and a Chimec Value 10 was recorded on the preceding day and 65 on the day after.

This example shows that Chimec Value, although it gives an indication as valid as MB, provides a larger difference, thus a higher sensibility, being, however, a simpler and more immediate test.

### Example 9 (Refinery Z)

This refinery was working an AMNA crude oil and an FR value 68 and a PV 1,05 were found, these indicating a relatively smooth run with respect to the tar stability. In spite of this the plant was fouling in an anomalous way.

By determining the Chimec Value in the same operating conditions, the presence of about 35 particles was shown. In the same refinery, working in another case a different oil (KIRKUK) the same FR and PV values were obtained on a non fouling run. The corresponding count of particles according to Chimec Value gave values of 8-10.

This example shows that the Chimec Value, whereas it gave an indication similar to the prior art as far as the tar stability was concerned, gave, however, a more precise indication with respect to the fouling rate of the plant.

## Claims

1. A method for determining the instantaneous reference parameter of tar stability defined as coke or fouling deposition tendency in a thermal conversion of oil treating plant, characterized by the following operations;
taking a drop of bottom residue obtained from the plant during operation with a determined furnace outlet temperature;
observing said drop under the microscope and counting the number of amorphous carbon particles having a size ranging from about 1 um up to 20 um, which are to be found within a pre-established area of the field of vision of the microscope; and
assuming said number of particles as instantaneous reference parameter of tar stability, to be compared with a standard index number chosen from a scale of reference index numbers determined in a blank test on each plant, for regulating the outlet temperature of the furnace to a higher or lower value, proportionate to the respectively negative or positive difference between said number of particles and the index number chosen for the running of the plant in pre-determined conditions.

2. Method according to claim 1, in which said field of the microscope is that visible to the eye with the microscope held still substantially at the center of a slide.

3. Method according to claim 1, in which said field of the microscope is that which results within the field of vision when running the microscope along a slide of standard length.

4. Method according to claim 2 or 3, in which said microscope has a lens of 20 to 500, preferably of 100 enlargements.

5. Method according to claim 1, in which said observation is carried out in part or totally automatically, by connecting a photographic apparatus to said microscope and effecting said counting on a picture taken by said photographic apparatus.

6. Method according to claim 1, in which said observation is carried out by connecting a telecamera to said microscope and observing enlarged images in said telecamera or on a monitor or display controlled by said telecamera.

7. Method according to claim 6, in which the output signals from said telecamera are fed to an electronic processor to manage the automation of said counting of the number of particles and distribution of the particles sizes through a specific software.

8. Method according to claim 7, in which the output from said electronic processor is connected to a system controlling the operating parameters of the plant, such as in particular the outlet furnace temperature, in order to control the parameters as a function of said counting, so that said system inserted in a production plant enables a continuous evaluation of the coke size to be made, by comparing this with the limit values in real time, whereby an immediate optimization of the process parameters can be obtained.

## Patentansprüche

1. Verfahren zur Bestimmung des momentanen Referenzparameters der Teerstabilität, definiert als Tendenz zur Ablagerung von Koks oder Schlamm, in einer thermischen Ölkonversionsalage, gekennzeichnet durch die folgenden Verfahrensschritte:
- Abnehmen eines Tropfens der Sumpf-Rückstände, die aus der Anlage bei Betrieb mit einer bestimmten Ofen-Auslaßtemperatur erhalten wurden;
- Anschauen des Tropfens unter dem Mikroskop und Zählen der Anzahl amorpher Kohlenstoff-Teilchen mit einer Größe im Bereich von 1 »m bis 20 »m, die innerhalb eines vorher festgelegten Bereichs des Blickfelds unter dem Mikroskop gefunden werden; und
- Annehmen der Zahl der Teilchen als momentaner Referenzparameter der Teerstabilität zum Vergleich mit einer Standard-Indexzahl, die gewählt ist aus einer Skala von Referenz-Indexzahlen, die in einem Blindversuch in jeder Anlage bestimmt werden, zum Regulieren der Ofen-Auslaßtemperatur auf einen höheren oder niedrigeren Wert proportional zu der jeweiligen negativen oder positiven Differenz zwischen der Zahl der Teilchen und der für den Betrieb der Anlage unter vorbestimmten Bedingungen gewählten Index-Zahl.

2. Verfahren nach Anspruch 1, worin das Blickfeld des Mikroskops dasjenige ist, das für das Auge sichbar ist, wobei das Mikroskop im wesentlichen im Zentrum eines Objektglases stillgehalten wird.

3. Verfahren nach Anspruch 1, wobei das Blickfeld des Mikroskops dasjenige ist, das im Blickfeld daraus resultiert, daß man das Mikroskop entlang einem Objektglas von Standard-Länge bewegt.

4. Verfahren nach Anspruch 2 oder Anspruch 3, worin das Mikroskop eine Linse mit einer 20- bis 500-facher Vergrößerung hat, vorzugsweise mit 100-facher Vergrößerung.

5. Verfahren nach Anspruch 1, worin das Anschauen teilweise oder vollständig automatisch erfolgt, indem man einen Photoapparat mit dem Mikroskop verbindet und den Zählvorgang an einem von dem Photoapparat aufgenommenen Bild durchführt.

6. Verfahren nach Anspruch 1, worin das Anschauen in der Weise erfolgt, daß man eine Fernsehkamera mit dem Mikroskop verbindet und vergrößerte Bilder in der Fernsehkamera oder auf einem Monitor oder einer Anzeige anschaut, die von der Fernsehkamera gesteuert werden.

7. Verfahren nach Anspruch 6, worin die Ausgangssignale der Fernsehkamera einer elektronischen Verarbeitungseinrichtung (Prozessor) zugeführt werden und so die Automatisierung des Zählens der Teilchenzahl und der Teilchengröße-Verteilung durch eine spezielle Software ermöglicht wird.

8. Verfahren nach Anspruch 7, worin die Ausgangssignale aus der elektronischen Verarbeitungseinrichtung (Prozessor) abgebende Leitung mit einem System verbunden ist, das die Betriebsparameter der Anlage wie beispielsweise insbesondere die Ofen-Auslaßtemperatur steuert, um die Parameter als Funktion des Zählvorgangs zu steuern, so daß das System bei Einbau in eine Produktionsanlage eine kontinuierliche Bewertung der Größe der entstehenden Koks-Teilchen ermöglicht, indem diese mit den Grenzwerten in Realzeit verglichen wird, wodurch eine unmittelbare Optimierung der Prozeßparameter erreicht werden kann.

## Revendications

1. Procédé pour déterminer le paramètre de référence instantané de la stabilité du goudron défini comme coke, ou la tendance au dépôt d'encrassement dans une installation de traitement du pétrole à conversion thermique, caractérisé par les opérations suivantes :
prélèvement d'une goutte de résidu de fond obtenue dans l'installation pendant le fonctionnement à une température de sortie de four déterminée ;
observer la goutte au microscope et compter le nombre de particules de carbone amorphe ayant une taille allant d'environ 1 »m jusqu'à 20 »m et qui sont rencontrées à l'intérieur d'une zone préétablie du champ de vision du microscope ; et
partir de ce nombre de particules comme paramètre de référence instantané de la stabilité du goudron à comparer avec un numéro d'indice standard choisi à partir d'une échelle de numéros d'indice de référence déterminée dans un essai à blanc sur chaque installation pour réguler la température de sortie du four à une valeur supérieure ou à une valeur inférieure, proportionnelle à la différence positive ou négative respectivement entre le nombre de particules et le nombre d'indice choisi pour la marche de l'installation dans des conditions prédéterminées.

2. Procédé selon la revendication 1, dans lequel le champ de vision du microscope est le champ visible à l'oeil avec le microscope maintenu immobilisé sensiblement au centre d'un porte-objet.

3. Procédé selon la revendication 1, dans lequel le champ du microscope est celui qui se situe dans le champ de vision lorsque l'on actionne le microscope le long d'un porte-objet de longueur standard.

4. Procédé selon la revendication 2 ou 3, dans lequel le microscope possède une lentille d'agrandissement de 20 à 500, de préférence 100.

5. Procédé selon la revendication 1, dans lequel l'observation s'effectue partiellement ou entièrement automatiquement en branchant un appareil photographique au microscope et en effectuant le comptage sur une image prélevée par l'appareil photographique.

6. Procédé selon la revendication 1, dans lequel l'observation s'effectue par branchement d'une télécaméra sur le microscope et en observant les images agrandies dans la télécaméra ou sur un moniteur ou écran d'affichage commandé par la télécaméra.

7. Procédé selon la revendication 6, dans lequel les signaux de sortie provenant de la télécaméra sont amenés à un processeur électronique pour diriger l'automatisation du comptage du nombre de particules et la répartition des tailles particulaires par un logiciel spécifique.

8. Procédé selon la revendication 7, dans lequel la sortie du processeur électronique est raccordée à un système commandant les paramètres de fonctionnement de l'installation en particulier la température du four de sortie pour commander les paramètres en fonction du comptage de sorte que le système incorporé dans une installation de production permet l'évaluation en continu de la taille du coke en comparant celle-ci aux valeurs limites en temps réel, permettant d'obtenir une optimisation immédiate des paramètres de traitement.
